# EUROPEAN PATENT SPECIFICATION

(11) **EP 1 141 358 B1**
(45) Date of publication and mention of the grant of the patent: **05.12.2007**
(21) Application number: 99962013.1
(22) Date of filing: 23.12.1999
(51) Int. Cl.: C12N 15/85, C12N 5/10, C12Q 1/68, A61K 35/00

(54) **STABLY TRANSFECTED CELLS FOR IDENTIFYING COMPOUNDS WHICH AFFECT STABILITY OF mRNA**
STABIELE TRANSFIZIERTE ZELLEN ZUM AUFFINDEN VON WIRKSTOFFEN, DIE DIE MRNA STABILITÄT BEEINFLUSSEN
CELLULES STABLEMENT TRANSFORMÉES PERMETTANT D'IDENTIFIER DES COMPOSÉS AFFECTANT LA STABILITE DE L'ARNm

(30) Priority: 24.12.1998 GB 9828709
(43) Date of publication of application: 10.10.2001
(73) Proprietor: Novation Pharmaceuticals Inc., New Westminster British Columbia V3M 1A7 (CA)
(72) Inventor: KASTELIC, Tania, Coquitlam, British columbia V3J 3B6 (CA); CHENEVAL, Dominique, Coquitlam, British Columbia V3J 3B6 (CA)
(74) Representative: Bassil, Nicholas Charles
(86) International application number: PCT/CA1999/001235
(87) International publication number: WO 2000/039314

(56) References cited:
- WO-A-93/20212
- WO-A-95/33831
- US-A- 5 731 343
- KASTELIC ET AL.: "INDUCTION OF RAPID IL-1BETA mRNA DEGRADATION IN THP-1 CELLS MEDIATED THROUGH THE AU-RICH REGION IN THE 3'UTR BY A RADICICOL ANALOGUE" CYTOKINE, vol. 8, no. 10, October 1996 (1996-10), pages 751-761, XP002138167 cited in the application
- BANHOLZER ET AL.: "RAPAMYCIN DESTABILIZES INTERLEUKIN-3 mRNA IN AUTOCRINE TUMOR CELLS BY A MECHANISM REQUIRING AN INTACT 3'UNTRANSLATED REGION" MOLECULAR AND CELLULAR BIOLOGY, vol. 17, no. 6, 1997, pages 3254-3260, XP002138168

## Description

This invention relates to a stably transfected cell line for use in the identification of biologically active compounds which have an effect on mRNA stability.

Recently, it has become increasingly apparent that the regulation of RNA half-life plays a critical role in the tight control of gene expression and that mRNA degradation is a highly controlled process. RNA instability allows for rapid up- or down-regulation of mRNA transcript levels upon changes in transcription rates. A number of critical cellular factors, e.g. transcription factors such as c-myc, or gene products which are involved in the host immune response such as cytokines, are required to be present only transiently to perform their normal functions. Transient stabilisation of the mRNAs which code for these factors permits accumulation and translation of these messages to express the desired cellular factors when required; whereas, under non-stabilised, normal conditions the rapid turnover rates of these mRNAs effectively limit and "switch off" expression of the cellular factors. However, abnormal regulation of mRNA stabilisation can lead to unwanted build up of cellular factors leading to undesirable cell transformation, e.g. tumour formation, or inappropriate and tissue damaging inflammatory responses.

Although the mechanisms which control mRNA stability are far from understood, sequence regions have been identified in a number of mRNAs, which appear to confer instability on the mRNAs which contain them. These sequence regions are referred to herein as "mRNA instability sequences". For example, typical mRNA instability sequences are the AREs (AU rich elements), which are found in the 3'UTR (3' untranslated region) of certain genes including a number of immediate early genes and genes coding for inflammatory cytokines, e.g. IL-1β and TNFα.

We have discovered compounds which promote instability of mRNAs which contain mRNA instability sequences. Such compounds may be used to induce degradation of mRNAs, thus preventing or reversing inappropriate mRNA accumulation and thereby decreasing or preventing unwanted protein, e.g. cytokine, expression. Thus such compounds are potentially useful pharmaceutically for prophylaxis or treatment of diseases or medical conditions which involve inappropriate mRNA stabilisation and accumulation and resultant undesirable protein expression.

The present invention relates to a stably transfected cell line for identifying compounds which affect the stability of mRNAs which contain mRNA instability sequences.

Accordingly the present invention provides a stably transfected cell line for use in screening for compounds to treat a disease of interest comprising:
(i) a reporter gene DNA expression system consisting of a reporter gene encoding a first protein which gives a detectable signal, a 5'UTR sequence and a 3'UTR sequence, wherein said 5'UTR sequence and said 3'UTR sequence comprise appropriate expression control elements, and a DNA sequence consisting of 20-100 nucleotides and comprising an mRNA instability sequence derived from a gene sequence related to said disease of interest and different from said reporter gene, wherein said DNA sequence is inserted into the 3'UTR sequence of said reporter gene; and
(ii) a control reporter gene DNA expression system comprising a reporter gene encoding a second protein which gives a detectable signal, a 5'UTR sequence and a 3'UTR sequence, wherein said 5'UTR sequence and said 3'UTR sequence comprise appropriate expression control elements, but lack any functional mRNA instability sequences; and wherein the cell line is derived from the native cell type in which said mRNA instability sequence is produced.

The stably transfected cell line may be used to screen individual compounds and libraries of compounds, including combinatorial compound libraries. It may be used as a first line screening assay to identify lead compounds and may be used to compare or quantify the mRNA instability promoting activity of compounds, e.g. to compare compounds produced from medicinal chemistry lead optimisation/derivatisation programmes.

Preferred embodiments of the invention are as defined in the claims.

Appropriate choice of promoter/enhancer sequences and other expression control sequences is a matter well within the ambit of the skilled worker in the art, and does not form a substantive part of the invention. Thus, for instance, for expression in mammalian cells a viral promoter such as an SV40, CMV or HSV-1 promoter may be used. On the other hand appropriate choice of mRNA instability sequence is of importance to the successful functioning of the reporter gene assay and forms part of the invention.

mRNA instability sequences have been identified in the UTRs, in particular the 3' UTRs, of a large number of transiently expressed genes including genes for cytokines, chemokines, nuclear transcription factors, protooncogenes, immediate early genes, cell cycle controlling genes, oxygenases, genes involved in and controlling of apoptosis. The natural RNA sequences which comprise the mRNA instability sequences are alternatively referred to as adenylate/uridylate (AU)-rich elements, or AREs. Transiently expressed genes which contain mRNA instability sequences include, for example, the genes coding for GM-CSF, *c-fos,* c-*myc,* c-*jun, krox*-20, *nur-*77*, zif*268, bc1-2, β-IFN, uPA, IL-1, IL-2, IL-3, IL-4, IL-5, IL-6, IL-7, IL-8, IL-9, IL-10, IL-11, IL-13, TNF-α, MCP1, syn1, β₂-AR, E-selectin, VCAM-1, ICAM-1, Gro-α, Gro-β, MMP-1, MMP-2, collagenases, P-glycoproteins (MDR), MRPs, Pγh1 (pf mdr), COXII, and MIP-2α.

The following publications include extensive discussion of mRNA instability sequences and AREs, the sequences motifs which they contain and (minimum) sequence requirements for mRNA destabilisation, as well as identifying a number of mRNA instability sequences and the genes which contain them:
Shaw & Kamen, Cell, Vol. 46, 659-667, August 29 1986 (GM-CSF);
Shyu et al., Genes & Development, 5:221-231 (1991) (*c-fos*);
Sachs, Cell, Vol. 74, 413-421, August 13 1993 (Review. "Messenger RNA Degradation in Eukaryotes");
Chen et al., Mol. Cell. Biol., Jan 1994, p 416-426 (*c-fos*);
Akashi et al., Blood, Vol. 83, No. 11, (June 1), 1994: pp 3182-3187 (GM-CSF etc.);
Nanbu et al., Mol. Cell. Biol., July 1994, p. 4920-4920 (Upa);
Stoecklin et al., J. Biol. Chem., Vol. 269, No. 46, November 18 1994, pp 28591-28597 (IL-3);
Lagnado et al., Mol. Cell. Biol., Dec. 1994, p. 7984-7995 (general);
Zhang et al., Mol. Cell. Biol., Apr. 1995, p. 2231-2244 (yeast);
Zubiaga et al., Mol. Cell. Biol., Apr. 1995, p. 2219-2230 (general);
Winstall et al., Mol. Cell. Biol., July 1995, p. 3796-3804 (*c-fos*, GM-CSF);
Chen et al., Mol. Cell. Biol., Oct. 1995, p. 5777-5788 (c-*fos*, GM-CSF);
Chen et al., TIBS 20 November 1995, 465-470 (review);
Levy et al., J. Biol. Chem., Vol. 271, No. %, February 2 1996, pp. 2746-2753 (VEGF);
Kastelic et al., Cytokine, Vol. 8, No. 10 (October), 1996: pp751-761;
Crawford et al., J. Biol. Chem., Vol. 272, No. 34, August 22 1997, pp. 21120-21127 (TNF-α);
Xu et al., Mol. Cell. Biol., Aug. 1997, Vol. 18, No. 8, p. 4611-4621 (general);
Danner et al., J. Biol. Chem., Vol.273, No. 6, February 6 1998, pp. 3223-3229 (human β₂-Adrenergic Receptor);
Lewis et al., J. Biol. Chem., Vol. 273, No. 22, May 29 1998, pp. 13781-13786 (TNF-α);
Chen, C.-Y. and Shyu, A.-B. Mol. Cell. Biol. Vol.14, No.12, 1994, pp. 8471-8482; and
Klausner, R. et al., Cell, Vol. 72, 1993, pp. 19-28.

As described in the above publications mRNA instability sequences often contain one or more copies of sequence motifs, e.g. selected from:
AUUUA; UAUUUAU; UUAUUUA(U/A)(U/A), and AUUUAUUUA.
Thus mRNA instability sequence for use in the invention usually contains at least 1, preferably at least 2, or more preferably at least 3 of such sequence motifs or parts thereof (e.g. normally containing at least 4 consecutive nucleotides from the motif) in appropriate juxtaposition, normally together, e.g. as tandem repeats, or with other, e.g. intervening, RNA sequences.

The mRNA instability sequence comprises from 20 up to 100 or more, preferably from about 30 to about 50, nucleotides in length. The mRNA instability sequence may be derived as a restriction fragment from the 3' UTR of an appropriate gene, or as a de novo synthesised nucleotide sequence. Alternatively the whole or a substantial part of the 3' UTR of an appropriate natural gene sequence, which contains a mRNA instability sequence may be used.

The mRNA instability sequence used is one derived from the mRNA which codes for a protein which is implicated in the disease of interest. Thus, for example, a mRNA instability sequence for use in detecting compounds which destabilise the mRNA which codes for a cytokine or oncogene which is involved in the aetiology of a particular disease process, is preferably derived from the gene which codes for the cytokine or oncogene in question, e.g. lead compounds for treatment of IL-1 mediated diseases, such as rheumatoid arthritis or osteoarthritis are preferably detected using a reporter gene expression system comprising an IL-1 mRNA instability sequence.

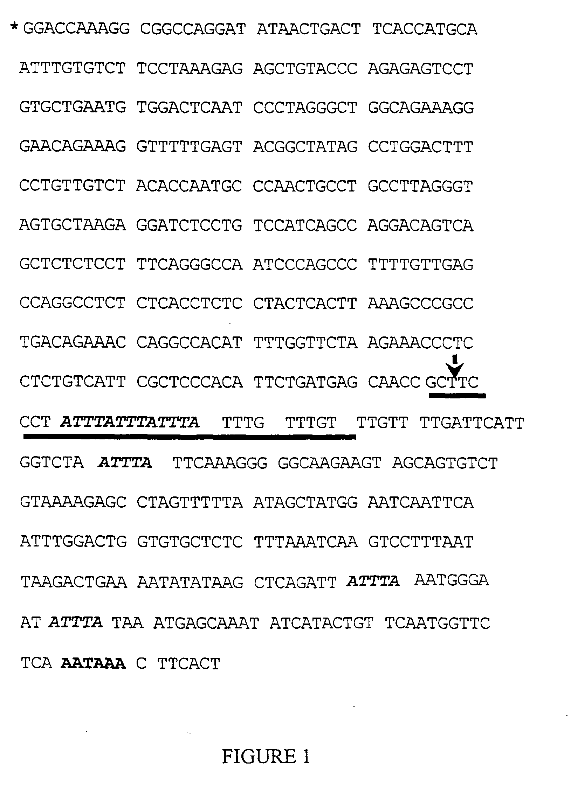

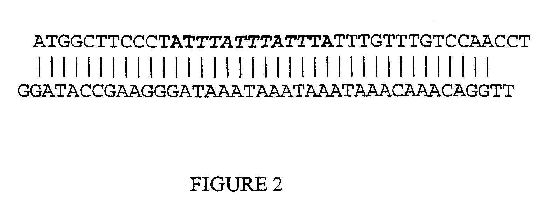

Thus by way of illustration of the invention a preferred mRNA instability sequence for use in the identification of compounds which destabilise IL-1β mRNA is derived from the 3' UTR of IL-1β mRNA, e.g. the sequence shown in Figure 1. More preferably the IL-1β mRNA instability sequence may comprise a fragment of the 3' UTR of IL-1β mRNA. For example, a particularly preferred IL-1β mRNA instability sequence comprises the 30 nucleotide sequence derived from the 3' UTR of IL-1β mRNA (shown in Figure 2).

The mRNA instability sequence is located in the 3' UTR of the reporter gene. Thus for example, the DNA sequence corresponding to the mRNA instability sequence is inserted as or as part of an appropriate DNA segment into a suitable restriction site in the 3' UTR of the native reporter gene.

The host cell is typically an eucaryotic host cell, in particular an animal host cell, especially a mammalian host cell.

The host cell is of the same general cell type as the cells which express the protein which is coded for by the mRNA which it is desired to destabilise. Thus for instance, if the assay of the invention is to be used for the identification of compounds which destabilise the mRNA coding for a cytokine, the host cell used is preferably a cell or cell line which is of the same or similar cell type to the cells which normally produce the cytokine in question. For example, monocyte or monocyte-like cell lines may be used as host cells for assaying for compounds which destabilise cytokine, e.g. IL-1β, mRNA. Preferred cell lines for oncogene and other cancer related gene mRNA instability assays are, e.g. Colon 205, KB 31, KB 8511, DU-145, HCT116, MCF7, MCF7/ADR, MDA-MB-231, MDA-MB-435 and MDA-MB-435/TO. Particularly preferred cell lines for use as the host cells in assays of the invention for identification of compounds which destabilise cytokine, e.g. IL-1β, mRNA are the THP-1 cell line (for instance as described by Auwerx J. (1991), Experientia, 47: 22-30) and similar monocytic, e.g. human leukaemia, cell lines.

The mRNA instability sequence and the host cell are derived from the native mRNA which it is desired to destabilise and the native cell type in which that mRNA is produced respectively. Thus for instance, for identification of compounds which destabilise cytokine mRNA, the mRNA instability sequence is preferably derived from the mRNA which codes for the cytokine in question and the host cell is preferably of the same cell type as the native cell type in which the cytokine mRNA is produced. For example, for identification of compounds which destabilise IL-1β mRNA, the mRNA instability sequence is preferably derived from the 3' UTR of IL-1β mRNA and the host cells used are monocyte-type cells, e.g. THP-1 cells.

Although the mechanism of mRNA destabilisation, and the role of mRNA instability sequences in this, is not fully understood, it is clear that the presence of other factors besides the destabilising compound and the mRNA instability sequence are required for mRNA destabilisation to take place; for instance, as discussed in previously identified literature references. Conveniently such other factors are provided by the transformed host cell environment and complement or complete the interaction of the compound and the mRNA instability sequence to effect destabilisation of the mRNA. Preferably the transformed host cells may be stimulated or otherwise activated to enhance mRNA destabilisation, e.g. to provided enhanced levels of the cellular factors required for mRNA destabilisation. In particular we have found that improved results are obtained in the assay of the invention if differentiated transformed host cells are used. For example, in the case of transformed THP-1 cells we have found that the best results are obtained if the transformed THP-1 cells are grown, differentiated and stimulated with γIFN and LPS as is normal for THP-1 cells, e.g. as described hereinafter in the Examples.

The protein coded by the reporter gene mRNA may itself comprise the detectable signal. For instance, the protein may comprise a fluorescent protein, e.g. green fluorescent protein. Preferably, however, the protein is such that it is capable of reacting with an appropriate substrate or other substance to give a detectable signal. Conveniently the protein coded by the mRNA is an enzyme or enzymically active fragment of an enzyme. Examples of suitable enzymes include horseradish peroxidase (HRP), chloramphenicol acetyltransferase (CAT), alkaline phosphatase (AP), secreted alkaline phosphatase (SEAP), β-galactosidase, or especially luciferase. Methods for detecting and determining such enzymes are well-known, using appropriate substrates and measurements; for instance, as described hereinafter for the determining the levels of luciferase expression. It will be appreciated, however, that any suitable detectable protein and measurement procedure may be used.

In the invention the presence of a compound which destabilises mRNA is indicated by a decrease in the magnitude of the detectable signal given by the protein produced from the expression system in the presence of the compound as compared with a control; destabilisation of the reporter gene mRNA by the compound leads to a decrease in expression of the protein and thus a decrease in the magnitude of the signal.

The invention is further described by way of illustration of the invention only in the following Examples which relate to a particular assay of the invention and refer to the accompanying Figures:
Figure 1 which shows the DNA sequence of IL-1β 3' UTR;
Figure 2 which shows the 30 bp fragment used as a mRNA instability sequence in Example 1;
Figure 3 which shows plasmid diagrams for pGL2_Neo30 and pGL2-Control;
Figure 4 which shows graphs of luciferase activity over the time of differentiation for clone No. 53 (A) and clone No. 63 (B);
Figure 5 shows graphs of luciferase half lives, 4 and 8 hours after addition of compounds for clones 53 and 63 treated with radicicol analog A (SDZ 216-732), actinomycin D (act D.) and cyclohexamide (CHX);
Figure 6 shows graphs of luciferase activity from clones 53 (solid bars) and 63 (open bars) treated with various concentrations of radicicol analog A (SDZ 216-732);
Figure 7 shows graphs of luciferase activity for undifferentiated (undiff) and differentiated (diff) clone 53 (solid bars) and clone 63(open bars) treated with radicicol analog A, and
Figure 8 shows a graph of the concentration inhibition of luciferase activity by radicicol analog A.

### EXAMPLES

We have shown earlier (Kastelic et al., CYTOKINE. Vol. 8, No. 10 (October), 1996: pp751-761) that radicicol analog A (the compound shown below) confers mRNA instability through the AU-rich element (ARE) motifs located in the 3' untranslated region (3' UTR) of genes subject to mRNA instability. For these studies, the segment of 3' UTR of IL-1β which contains all the AREs was deleted and the resulting IL-1β -AU cDNA was subcloned into an expression vector. Stably transfected THP-1 cells containing this construct were analyzed by the RNase protection method (Kastelic et al. ibid) and showed resistance of the AU-less derived RNA towards radicicol analog A.

The 3'UTR of IL-1β mRNA contains a total of 6 AUUUA motifs three of which are in tandem (see Figure 1). For the construction of the luciferase reporter gene assay, we used only the a fragment comprising of the underlined sequence shown in Fig. 1 which contains three tandem repeats. Findings by Zubiaga et al (ibid) indicate that the minimal sequence of the mRNA instability motif is UUAUUUAUU (a sequence which occurs in the inserted 30 bp IL-1β fragment which we used) rather than just AUUUA alone.

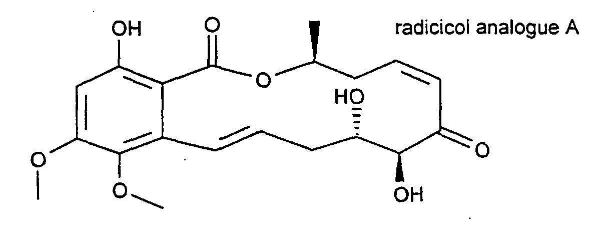

### Example 1: Construction of pGL2 Neo30 and stable cell lines

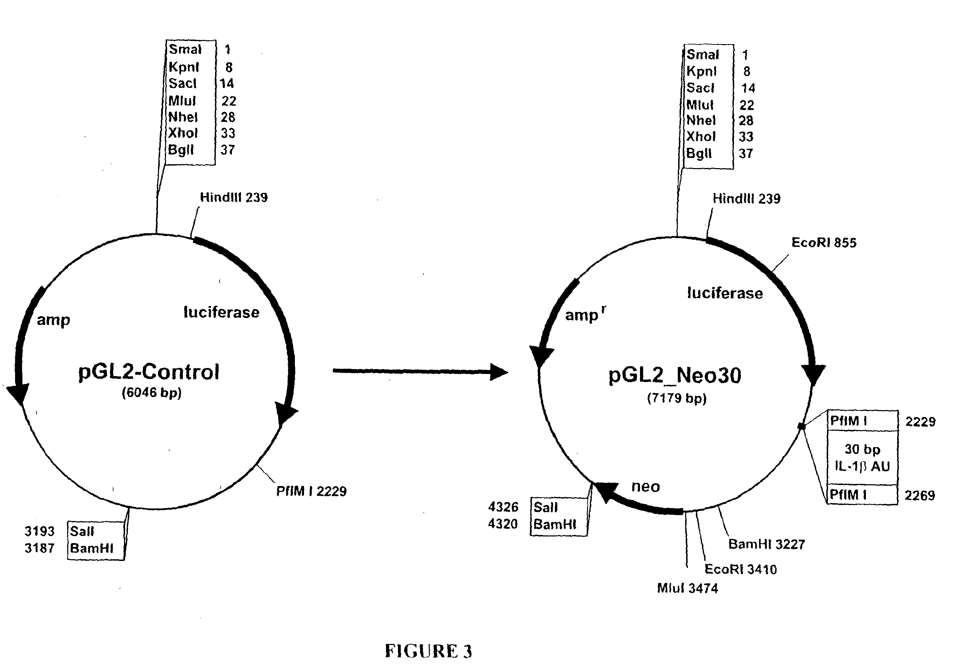

In order to obtain a vector for stable integration into THP-1 cells, a XhoI - SalI fragment of the neo resistant gene (expressing aminoglycoside 3' phosphotransferase) derived from pMCIneo (Stratagene) is subcloned into the SalI site of pGL2-Control (Promega). This resulting plasmid was called pGL2_Neo. A 30bp fragment (containing three tandem AUUUA motifs, based on the IL-Iβ 3'UTR sequence) obtained by annealing two complementary synthetic oligonucleotides (see Figure 2) is subcloned into pGL2_Neo using the Pf1M1 restriction site. This results in the luciferase expression vector pGL2_Neo30 (Fig. 3). Fig. 2 shows the IL-1β 3'UTR sequence containing three tandem AUUUA motifs used for ligation into the Pf1MI site of pGL2_Neo. Expression vector pGL2-β-galactosidase (Promega) has the *lacZ* gene driven by the same promoter (SV40) as the luciferase gene in pGL2_Neo30 and pGL2_Neo, but plasmid pGL2-β-galactosidase does not contain any mRNA instability sequences.

THP-1 cells are then transfected with pGL2_Neo vector (to generate control cell lines) or are cotransfected with pGL2_Neo30 vector pGL2-β-galactosidase by electroporation. 10⁷ cells/ml in 1.3mM KH₂P0₄, 7.36m.M Na₂HP0₄, 2.44mM KCl, 124mm NaCl, 5mM glucose, 9.6µM MgCl₂ and 16µM CaCI₂ pH 7.2 are transfected with 20µg of DNA in a Bio-Rad Gene Pulser (250V, 690µF and indefinite resistance) using a 0.4cm cuvette. Cells are subsequently cultured in RPMI medium containing 10%FBS, 2mM L-Gln (L-glutamine), 50µM 2-mercaptoethanol and 600µg/ml of G418 (geneticin). After transfection of pGL2_Neo30 and pGL2_Neo into THP-1 cells, stable cell lines are obtained by selection for G418 resistance and assayed for luciferase activity (and the cotransfected cell line is also assayed for β-galactosidase activity which can serve as an internal control - see Example 5 below). One cell line of each transfection is chosen for further analysis; the pGL2_Neo30/ pGL2-β-galactosidase cell line is referred to as clone No. 63 and the pGL2_Neo cell line as clone No. 53. No endogenous luciferase activity could be detected in normal THP-1 cells.

The tissue culture and luciferase activity measurements are carried out as described below.

### Tissue culture:

The transfected human monocytic leukaemia cell lines, clones No. 53 and 63 are grown in RPMI medium supplemented with 110 U/ml penicillin, 100 µg/ml streptomycin, 2 mM L-Gln and 2 g/l NaHCO₃. Heat-treated FBS (5%) is added before use. The cells are grown to a density of 5x 10⁵/ml and induced to differentiate with 100 U/ml (final concentration) γIFN. Three hours later, 10 µl of LPS (5µg/ml final concentration) is added. This time point is designated time 0. Compounds are added at various times after LPS addition as indicated.

### Luciferase activity measurement:

In order to adapt the system to the use of 96 well plates, cells are grown in Packard flat bottom white polystyrene microplates (Cat. No.6005180) in RPM1 medium lacking phenol red (AMIMED). Cells are plated at 5x10⁴/well. After treatment of the cells, luciferase is measured using the Packard Luc Lite system (Cat. No.601691 1) according to the manufacturer's instructions in a final volume of 205µl. Briefly, to a cell suspension of 5 x 10⁵ cells/ml, γIFN (1000U/ml Boehringer Mannheim No. 1050494) to a final concentration of 100 U/ml and 0.25% (v/v) Luc Lite Enhancer is added. After a 3 hour incubation LPS (50µg/ml SIGMA L-8274) is added to give 5µg/ml final concentration. The cells are then plated at 5x10⁴/100µl/well into flat bottom white polystyrene microplates (Packard, Cat. No. 6005180) and incubated for 16 hours. 5 µl of compound solution or control vehicle is then added and the cells are further incubated as indicated. 100 µl of luciferase substrate solution is added and the plates are covered with TopSeal-A press-on adhesive sealing film (Packard Cat.No. 6005185) before measuring luminescence with a Packard Top Count Scintillation Counter at 22°C. The luciferase signal is stable for at least 90 min.

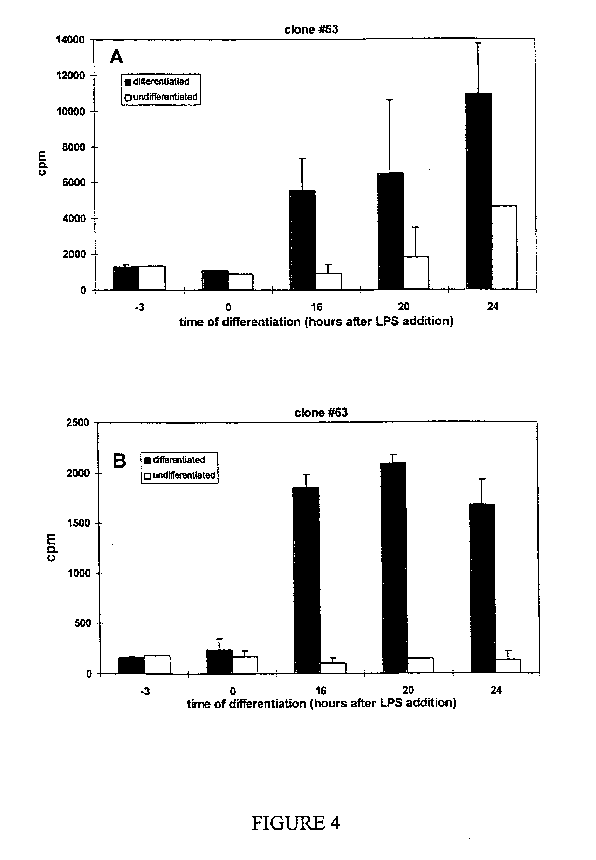

The differentiation-dependent induction of luciferase activity in the two cell lines, Nos. 53 (A) and 63 (B) are tested and the results obtained are shown in Figs. 4 A and B. In both clones a distinct induction of luciferase expression can be observed, maintaining high levels of activity throughout the time of the assay. This elevated and constant expression of luciferase should be born in mind when analyzing effects of compounds inducing mRNA instability. mRNA degradation will be in constant competition with de novo transcription, unlike the situation in wild-type THP-1 cells were in the case of IL-1β-mRNA, highest levels are obtained 16 hours after LPS addition. One would expect in the case of luciferase to see a weaker effect of mRNA destabilizing drugs since transcription remains high. Indeed this is what we observe in the case of radicicol analog A, see below.

### Example 2: Half life of Luciferase mRNA and protein

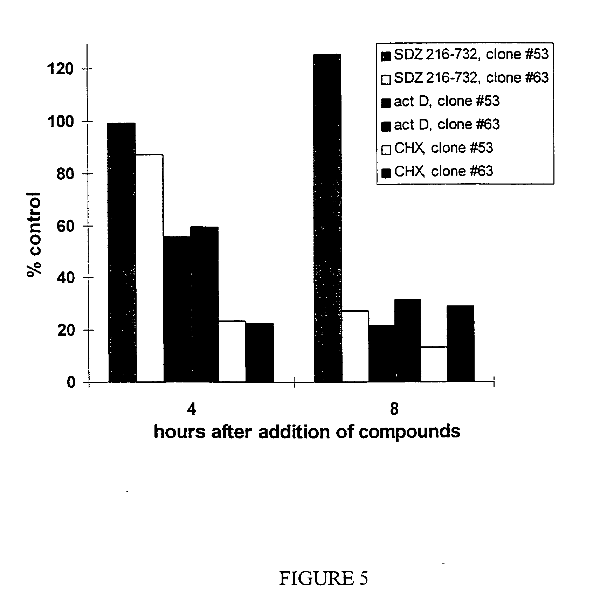

To measure mRNA degradation using luciferase protein activity it is important to know the half life of the luciferase enzyme in order to determine an optimal time for assaying for potential mRNA destabilizing agents by way of luciferase protein stability. The possibility exists that mRNA could be degraded but due to a long half life of the protein, high enzyme activities could persist. Therefore we analyzed luciferase activities after addition of the transcriptional inhibitor actinomycin D (act. D) or the translational inhibitor cycloheximide (CHX). Fig. 5 shows that in the presence of 20µg/ml act.D as well as in the presence of 20 µM CHX, luciferase activities rapidly decline and after 8 hours of incubation reach a level comparable to the inhibition achieved by radicicol analog A. In view of this relatively short half life of the luciferase enzyme, it is safe to assess any substance for activity on mRNA degradation as early as 8 hours after compound addition.

### Example 3: Effect of the radicicol analog A.

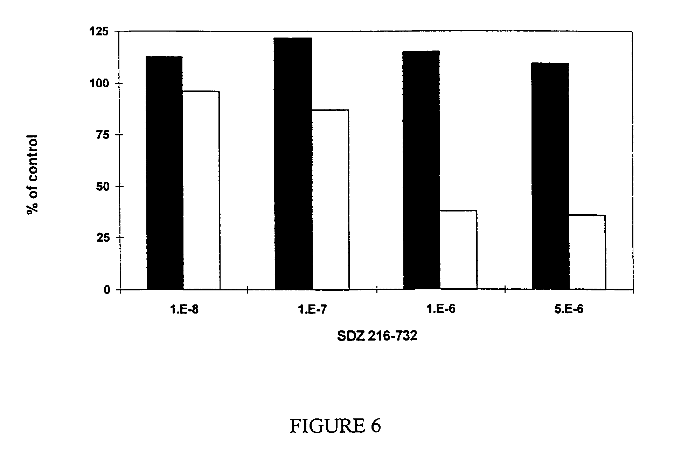

The THP-1 cell lines, clone No. 63 (containing pGL2_Neo30) and clone No. 53 (containing pGL2-Neo) are grown, differentiated with γIFN and stimulated with LPS identical to normal THP-1 cells. Radicicol analog A is added 16 hours after the addition of LPS and cell extracts are then taken 8 hours later or as indicated. Luciferase activity is inhibited by 1 µM radicicol analog A on average by 50% +/-17%, in some cases inhibition was as great as 93%, whereas up to 5x10⁻⁶M of radicicol analog A has no effects on the control clone No. 53, Fig. 6 (solid bars indicate clone No. 53, open bars clone No. 63).

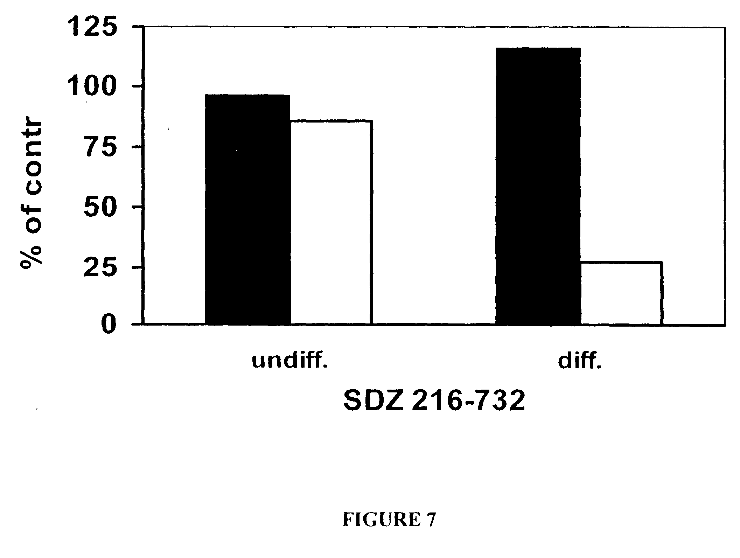

Interestingly, undifferentiated (undiff) clone No. 63 (open bars) when treated with radicicol analog A showed only a limited reduction of luciferase activity (Fig. 7, solid bars indicate clone No. 53), which is either due to the lower expression of luciferase or is indicative of the involvement of a differentially expressed or modified component in the mRNA degradation process mediated by AU-rich elements. Indeed, gel retardation experiments using 241 bp of the AU-rich 3' UTR of IL-1β as a riboprobe showed the binding of additional proteins with γIFN induced differentiation or modification (not shown).

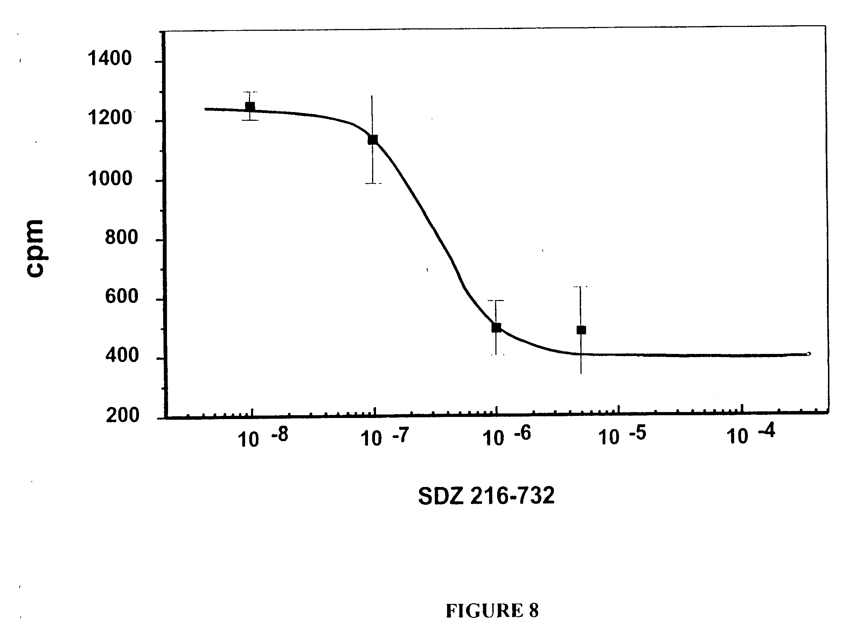

Concentration dependent inhibition of luciferase activity is shown in Fig. 8. Concentrations of radicicol analog A higher than 5x10⁻⁶ M also inhibited the control clone due to cytotoxicity or inhibitory activity on transcription.

### Example 4: Application of assay to a number of selected substances

A number of selected substances are tested for their activity in the assay of the invention substantially as described in Example 3 (for differentiated cells). The results obtained are given in the Table 1 below. Radicicol (see formula II below) and radicicol analog A show a clear effect on mRNA stability; other compounds tested did not show activity in the assay used.

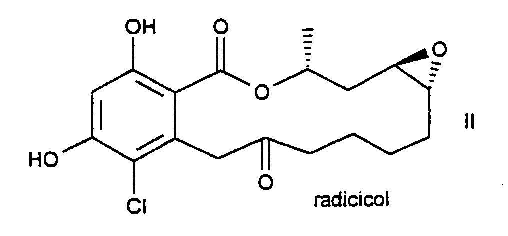

**TABLE 1**

| COMPOUND | Luciferase activity (% of control) | |
|---|---|---|
| | clone No. 53 | clone No. 63 |
| peptidic ICE inhibitor | 87 | 104 |
| stemphon | 95 | 90 |
| radicicol | 98 | 47 |
| (17α)-23-(E)-dammara-20,23-dien-3β,25-diol | 116 | 91 |
| radicicol analog A | 120 | 49 |
| thalidomide | 98 | 112 |
| dexamethasone | 72 | 63 |
| cyclosporin A | 82 | 74 |

### Example 5: Application of assay using a single cell line

In the previous examples, test compounds are assayed by comparing their activity in two separate cell lines (clone 53 and clone 63). However, clone 63 was cotransfected with two separated plasmids: one plasmid (pGL2_Neo30) contains the luciferase gene with the 30 bp instability sequence driven by the SV40 promoter and the other plasmid (pGL2-β-galactosidase) contains the *lacZ* gene driven by the SV40 promoter but contains no mRNA instability sequences. The β-galactosidase activity of this cell line should not be effected by exposure of the cells to compounds which promote mRNA instability via mRNA instability sequences. As a result, one should be able (in theory) to screen for compounds having mRNA instability activity by simply comparing luciferase activity in unstimulated cells versus stimulated cells and comparing the β-galactosidase activity in these same cells. To test this hypothesis, the effect of radicicol analog A on luciferase activity and β-galactosidase activity in clone 63 (stimulated and unstimulated cells) was compared to the effect of radicicol analog A on stimulated and unstimulated cells of clone 63 and clone 53. The assay was performed as described in the previous Examples. Table 2 shows the luciferase activities of various concentrations of radicicol analog A in γIFN/LPS stimulated and unstimulated cells of clones 63 and 53. Activities are given in % of control and are based on means of three independent experiments controlled for cell numbers. Table 3 shows the β-galactosidase activities in stimulated and unstimulated cells of clone 63. Activities are given in % of control and are based on means of three independent experiments controlled for cell numbers. It is clear from the data that both the assay of Table 2 and that of Table 3 would have identified radicicol analog A as an active compound.

**TABLE 2**

| **Luciferase activity** | | | | |
|---|---|---|---|---|
| | **clone 63** | | **clone 53** | |
| | unstimulated | γIFN/LPS stimulated | unstimulated | γIFN/LPS stimulated |
| | (%control) | (%control) | (%control) | (%control) |
| No compound | 100 | 100 | 100 | 100 |
| 1 µM radicicol analog A | 63 | 7 | nd | 88 |
| 10 µM radicicol analog A | 11 | 2 | 87 | 63 |

**TABLE 3**

| **β-galactosidase activity** | | | | |
|---|---|---|---|---|
| | **clone 63** | | **clone 53** | |
| | unstimulated | γIFN/LPS stimulated | unstimulated | γIFN/LPS stimulated |
| | (%control) | (%control) | (%control) | (%control) |
| No compound | 100 | 100 | 100 | 100 |
| 1 µM radicicol analog A | 96 | 97 | 99 | 98 |
| 10 µM radicicol analog A | 84 | 70 | 103 | 62 |

## Claims

1. A stably transfected cell line for use in screening for compounds to treat a disease of interest comprising:
i) a reporter gene DNA expression system consisting of a reporter gene encoding a first protein which gives a detectable signal, a 5'UTR sequence and a 3'UTR sequence, wherein said 5'UTR sequence and said 3'UTR sequence comprise appropriate expression control elements, and a DNA sequence consisting of 20-100 nucleotides and comprising an mRNA instability sequence derived from a gene sequence related to said disease of interest and different from said reporter gene, wherein said DNA sequence is inserted into the 3'UTR sequence of said reporter gene; and
ii) a control reporter gene DNA expression system comprising a reporter gene encoding a second protein which gives a detectable signal, a 5'UTR sequence and a 3'UTR sequence, wherein said 5'UTR sequence and said 3'UTR sequence comprise appropriate expression control elements, but lack any functional mRNA instability sequences; and
wherein the cell line is derived from the native cell type in which said mRNA instability sequence is produced.

2. The transfected cell line according to claim 1, wherein said mRNA instability sequence is derived from genes coding for cytokines, chemokines, nuclear transcription factors, protooncogenes, immediate early genes, cell cycle controlling genes, oxygenases, or genes involved in and controlling of apoptosis.

3. The transfected cell line according to claim 1, wherein said mRNA instability sequence is derived from genes coding for GM-CSF, c-fos, c-myc, c-jun, krox-20, nur-77, zif268, bc1-2, β-IFN, uPA, IL-1, IL-2, IL-3, IL-4, IL-5, IL-6, IL-7, IL-8, IL-9, IL-10, IL-11, IL-13, TNF-α, MCP-1, syn1, β2-AR, E-selectin, VCAM-1, ICAM-1, Gro-α, GRo-β, MMP-1, MMP-2, collagenases, P-glycoproteins (MDR), MRPs, Pγh1 (PF mdr), COXII, or MIP-2α.

4. The transfected cell line according to claim 1, wherein said mRNA instability sequence is derived from a cancer-related gene and the cell line is derived from Colon 205, KB31, KB8511, DU145, HCT116, MCF7, MCF7/ADR, MDA-MB-231, MDA-MB-435, or MDA-MB435/TO.

5. The transfected cell line according to claim 1, wherein said mRNA instability sequence is derived from a gene encoding a cytokine and the cell line is derived from a monocyte or monoctye-like cell line.

6. The transfected cell line according to claim 5, wherein said mRNA instability sequence is a 30 nucleotide fragment of the IL-1β 3'UTR sequence and said cell line is derived from THP-1.

7. The transfected cell line according to any one of claims 1-6, wherein said first protein which gives a detectable signal is a fluorescent protein or an enzyme.

8. The transfected cell line according to claim 7, wherein said enzyme is luciferase.

9. The transfected cell line according to claim 6, wherein said first protein which gives a detectable signal is luciferase and said second protein which gives a detectable signal is β-galactosidase.

10. An assay system for screening for compounds to treat a disease of interest comprising the transfected cell line according to any one of claims 1-9.

11. Use of the transfected cell line according to any one of claims 1-9 for screening for compounds to treat a disease of interest.

## Patentansprüche

1. Stabil transfizierte Zelllinie zur Verwendung beim Screenen auf Verbindungen zur Behandlung einer interessierenden Krankheit, umfassend:
i) ein Reporter-Gen-DNA-Expressionssystem bestehend aus einem Reporter-Gen, welches für ein erstes Protein codiert, das ein detektierbares Signal abgibt, einer 5'UTR-Sequenz und einer 3'UTR-Sequenz, wobei die 5'UTR-Sequenz und die 3'UTR-Sequenz geeignete Expressionskontrollelemente umfassen, und einer DNA-Sequenz, die aus 20-100 Nukleotiden besteht und eine mRNA-Instabilitätssequenz aufweist, welche aus einer Gen-Sequenz stammt, die mit der interessierenden Krankheit in Verbindung steht und sich vom Reporter-Gen unterscheidet, wobei die DNA-Sequenz in die 3'UTR-Sequenz des Reporter-Gens insertiert ist; und
ii) ein Kontroll-Reporter-Gen-DNA-Expressionssystem, umfassend ein Reporter-Gen, welches für ein zweites Protein codiert, das ein detektierbares Signal abgibt, eine 5'UTR-Sequenz und eine 3'UTR-Sequenz, wobei die 5'UTR-Sequenz und die 3'UTR-Sequenz geeignete Expressionskontrollelemente umfassen, jedoch keinerlei funktionelle mRNA-Instabilitätssequenzen aufweisen; und
wobei die Zelllinie vom nativen Zelltyp stammt, in welchem die mRNA-Instabilitätssequenz erzeugt wird.

2. Transfizierte Zelllinie nach Anspruch 1, wobei die mRNA-Instabilitätssequenz von Genen stammt, die für Cytokine, Chemokine, Kern-Transkriptionsfaktoren, Protoonkogene, immediate early-Gene, Zellzyklus-kontrollierenden Gene, Oxygenasen oder Gene, die an der Apoptose beteiligt sind und diese steuern, codieren.

3. Transfizierte Zelllinie nach Anspruch 1, wobei die mRNA-Instabilitätssequenz von Genen stammt, die für GM-CSF, c-fos, c-myc, c-jun, krox-20, nur-77, zif268, bcl-2, β-IFN, uPA, IL-1, IL-2, IL-3, IL-4, IL-5, IL-6, IL-7, IL-8, IL-9, IL-10, IL-11, IL-13, TNF-α, MCP-1, syn1, β2-AR, E-Selektin, VCAM-1, ICAM-1, Gro-α, GRo-β, MMP-1, MMP-2, Collagenasen, P-Glycoproteine (MDR), MRPs, Pγh1 (PF mdr), COXII oder MIP-2α codieren.

4. Transfizierte Zelllinie nach Anspruch 1, wobei die mRNA-Instabilitätssequenz von einem mit Krebs verwandten Gen stammt und die Zelllinie von Colon 205, KB31, KB8511, DU145, HCT116, MCF7, MCF7/ADR, MDA-MB-231, MDA-MB-435 oder MDA-MB435/TO stammt.

5. Transfizierte Zelllinie nach Anspruch 1, wobei die mRNA-Instabilitätssequenz von einem Gen stammt, das für ein Cytokin codiert, und die Zelllinie von einer Monozyten- oder Monozytenartigen Zelllinie stammt.

6. Transfizierte Zelllinie nach Anspruch 5, wobei die mRNA-Instabilitätssequenz ein 30-Nukleotid-Fragment der IL-1β3'UTR-Sequenz ist und die Zelllinie von THP-1 stammt.

7. Transfizierte Zelllinie nach einem der Ansprüche 1 bis 6, wobei das erste Protein, welches ein detektierbares Signal abgibt, ein fluoreszierendes Protein oder ein Enzym ist.

8. Transfizierte Zelllinie nach Anspruch 7, wobei das Enzym Luciferase ist.

9. Transfizierte Zelllinie nach Anspruch 6, wobei das erste Protein, welches ein detektierbares Signal abgibt, Luciferase ist, und das zweite Protein, welches ein detektierbares Signal abgibt, β-Galactosidase ist.

10. Untersuchungssystem zum Screenen auf Verbindungen zur Behandlung einer interessierenden Krankheit, umfassend die transfizierte Zelllinie nach einem der Ansprüche 1 bis 9.

11. Verwendung der transfizierten Zelllinie nach einem der Ansprüche 1 bis 9 zum Screenen auf Verbindungen zur Behandlung einer interessierenden Krankheit.

## Revendications

1. Lignée cellulaire transfectée de manière stable pour une utilisation dans le criblage de composés pour le traitement d'une maladie d'intérêt comprenant :
i) un système d'expression d'ADN de gène rapporteur comprenant un gène rapporteur codant pour une première protéine qui donne un signal détectable, une séquence UTR en 5' et une séquence UTR en 3', où ladite séquence UTR en 5' et ladite séquence UTR en 3' comprennent des éléments appropriés de contrôle de l'expression et une séquence d'ADN comprenant 20-100 nucléotides et comprenant une séquence d'instabilité de l'ARNm provenant d'une séquence d'un gène associée à ladite maladie d'intérêt et différente dudit gène rapporteur, où ladite séquence d'ADN est insérée dans la séquence UTR en 3' dudit gène rapporteur; et
ii) un système d'expression d'ADN de gène rapporteur témoin comprenant un gène rapporteur codant pour une deuxième protéine qui donne un signal détectable, une séquence UTR en 5' et une séquence UTR en 3', où ladite séquence UTR en 5' et ladite séquence UTR en 3' comprennent des éléments appropriés de contrôle de l'expression mais ne présentent pas de quelconques séquences fonctionnelles d'instabilité de l'ARNm; et
dans laquelle la lignée cellulaire provient du type cellulaire natif dans lequel ladite séquence d'instabilité de l'ARNm est produite.

2. Lignée cellulaire transfectée suivant la revendication 1, dans laquelle ladite séquence d'instabilité de l'ARNm provient de gènes codant pour des cytokines, des chimiokines, des facteurs nucléaires de transcription, des protooncogènes, des gènes précoces immédiats, des gènes de contrôle du cycle cellulaire, des oxygénases ou des gènes impliqués dans l'apoptose et la contrôlant.

3. Lignée cellulaire transfectée suivant la revendication 1, dans laquelle ladite séquence d'instabilité de l'ARNm provient de gènes codant pour les GM-CSF, c-fos, c-myc, c-jun, krox-20, nur-77, zif268, bcl-2, β-IFN, uPA, IL-1, IL-2, IL-3, IL-4, IL-5, IL-6, IL-7, IL-8, IL-9, IL-10, IL-11, IL-13, TNF-α, MCP-1, syn1, β2-AR, E-selectine, VCAM-1, ICAM-1, Gro-α, Gro-β, MMP-1, MMP-2, des collagénases, des P-glycoprotéines (MDR), des MRP, Pγh1 (PF mdr), COXII ou MIP-2α.

4. Lignée cellulaire transfectée suivant la revendication 1, dans laquelle ladite séquence d'instabilité de l'ARNm provient d'un gène associé à un cancer et la lignée cellulaire provient des Colon 205, KB31, KB8511, DU145, HCT116, MCF7, MCF7/ADR, MDA-MB-231, MDA-MB-435 ou MDA-MB435/TO.

5. Lignée cellulaire transfectée suivant la revendication 1, dans laquelle ladite séquence d'instabilité de l'ARNm provient d'un gène codant pour une cytokine et la lignée cellulaire provient d'un monocyte ou d'une lignée cellulaire de type monocyte.

6. Lignée cellulaire transfectée suivant la revendication 5, dans laquelle ladite séquence d'instabilité de l'ARNm est un fragment de 30 nucléotides de la séquence UTR en 3' de l'IL-1β et ladite lignée cellulaire provient des THP-1.

7. Lignée cellulaire transfectée suivant l'une quelconque des revendications 1-6, dans laquelle ladite première protéine qui donne un signal détectable est une protéine fluorescente ou une enzyme.

8. Lignée cellulaire transfectée suivant la revendication 7, dans laquelle ladite enzyme est la luciférase.

9. Lignée cellulaire transfectée suivant la revendication 6, dans laquelle ladite première protéine qui donne un signal détectable est la luciférase et ladite deuxième protéine qui donne un signal détectable est la β-galactosidase.

10. Système d'analyse pour le criblage de composés pour le traitement d'une maladie d'intérêt comprenant la lignée cellulaire transfectée suivant l'une quelconque des revendications 1-9.

11. Utilisation de la lignée cellulaire transfectée suivant l'une quelconque des revendications 1-9 pour le criblage de composés pour le traitement d'une maladie d'intérêt.
